# EUROPEAN PATENT APPLICATION

(11) **EP 4 803 027 A2**
(43) Date of publication of application: **09.09.2026**
(21) Application number: 26162065.2
(22) Date of filing: 03.03.2026
(51) Int. Cl.: A61B 34/10

(54) **MEDICAL DEVICE AND OPERATION METHOD THEREOF**

(30) Priority: 04.03.2025 JP 2025033504
(71) Applicant: FUJIFILM Corporation, Tokyo 106-8620 (JP)
(72) Inventor: MACHII, Yusuke, Tokyo, 106-8620 (JP)
(74) Representative: Dehns Germany Partnerschaft mbB

(57) **Abstract**

The present disclosure provides a medical device (12) and an operation method thereof that can display whether resection of a target to be resected has or has not been completed without requiring a user to take any time and effort.

A 3D organ model acquisition unit (20) acquires a 3D organ model (27) corresponding to an observation target organ (27a) to be observed. A camera video acquisition unit (21) acquires a laparoscopic video as a camera video. A first resection information generation unit (23) recognizes resection of the observation target organ from the laparoscopic video, and outputs first resection information including whether the resection has or has not been completed at a first resection timing. A display controller (22) performs display related to whether the resection has or has not been completed in the 3D organ model based on the first resection information.

## Description

### BACKGROUND OF THE INVENTION

### 1. Field of the Invention

The present disclosure relates to a medical device used for surgery in an abdominal cavity, such as in laparoscopic procedures, and an operation method thereof.

### 2. Description of the Related Art

In surgery of an organ, it is important to clarify which part is to be resected. For example, in a case of surgery of a liver, as in JP2012-34988A (corresponding to US2013/0144160A1), a dominant region of the liver dominated by blood vessels is determined as a part to be resected from a three-dimensional functional image, and the part to be resected is clearly defined by assigning different colors to the dominant region and other regions.

### SUMMARY OF THE INVENTION

As described above, in the surgery of the organ, it is important to understand whether or not blood vessels or lesions, which are scheduled to be resected in a preoperative plan for the organ resection, have already been resected in the surgery in order to determine whether the surgery is proceeding correctly according to the preoperative plan, or which blood vessel or lesion should be resected next. On the other hand, in a 3D organ model, in a case where the resection of the blood vessels or lesions to be resected is completed, manual switching to a hidden display state is performed. However, manually switching the display is time-consuming and places a burden on a user.

An object of the present disclosure is to provide a medical device and an operation method thereof that can display whether resection of a target to be resected has or has not been completed without requiring a user to take any time and effort.

According to an aspect of the present invention, there is provided a medical device comprising: a processor, in which the processor acquires a 3D organ model corresponding to an observation target organ to be observed, acquires a camera video, recognizes resection of the observation target organ from the camera video and outputs first resection information including whether the resection has or has not been completed at a first resection timing, and performs display related to whether the resection has or has not been completed in the 3D organ model based on the first resection information.

It is preferable that the processor estimate a scheduled resection target that is scheduled to be resected at a second resection timing after the first resection timing from the 3D organ model and the first resection information, and output the scheduled resection target as second resection information, and perform either the display related to whether the resection has or has not been completed in the 3D organ model and display related to the scheduled resection target based on the second resection information in addition to the first resection information, or display related to the scheduled resection target in the 3D organ model based on the second resection information instead of the first resection information.

It is preferable that the processor estimate a scheduled resection target that is scheduled to be resected at a second resection timing after the first resection timing from predetermined resection plan information and the first resection information, and output the scheduled resection target as second resection information, and perform either the display related to whether the resection has or has not been completed in the 3D organ model and display related to the scheduled resection target based on the second resection information in addition to the first resection information, or display related to the scheduled resection target in the 3D organ model based on the second resection information instead of the first resection information.

It is preferable that the processor estimate, from the camera video, a pose transformation matrix (a posture matrix) in a Viewer coordinate system that displays the 3D organ model, the posture matrix representing a spatial pose (a posture) of the observation target organ, and perform the display related to whether the resection has or has not been completed in the 3D organ model and display related to the posture of the observation target organ based on the posture matrix in addition to the first resection information.

It is preferable that the display related to whether the resection has or has not been completed in the 3D organ model be display for distinguishing between completed resection and incomplete resection in the 3D organ model. It is preferable that, of the observation target organ, a resection cross section that has been resected be displayed as the completed resection, and a non-resected portion be displayed as the incomplete resection. It is preferable that, among blood vessels included in the observation target organ, a resected blood vessel be displayed as the completed resection, and a blood vessel that has not been resected but is scheduled to be resected be displayed as the incomplete resection. It is preferable that, for a lesion included in the observation target organ, a lesion that has not been resected be displayed as the incomplete resection, and a resected lesion be displayed as the completed resection.

It is preferable that the display related to the scheduled resection target in the 3D organ model be display for distinguishing between the scheduled resection target and other portions in the observation target organ. It is preferable that a resection cross section to be resected at the second resection timing in the observation target organ be displayed as the scheduled resection target. It is preferable that a blood vessel to be resected at the second resection timing among blood vessels included in the observation target organ be displayed as the scheduled resection target. It is preferable that, for a lesion included in the observation target organ, a lesion to be resected at the second resection timing be displayed as the scheduled resection target.

It is preferable that the camera video be obtained from videos from a plurality of cameras. It is preferable that the processor output the first resection information based on an ultrasound video in addition to the camera video.

It is preferable that the processor output the first resection information for each blood vessel included in the observation target organ. It is preferable that the processor output the first resection information for each lesion included in the observation target organ. It is preferable that the processor output the first resection information based on correspondence information indicating a correspondence relationship between an anatomical structure in the camera video and a structure in the 3D organ model in addition to the camera video. It is preferable that the processor reconstruct an intraoperative 3D organ model of the observation target organ from the camera video or an ultrasound video, and the correspondence relationship be calculated from the intraoperative 3D organ model and the 3D organ model.

It is preferable that the processor output the first resection information based on the posture matrix in addition to the camera video. It is preferable that the processor output notification information based on the camera video and the second resection information. It is preferable that the processor output plan change information based on the camera video and the second resection information, and correct the resection plan information based on the plan change information.

According to another aspect of the present invention, there is provided an operation method of a medical device, the operation method comprising: a step of acquiring a 3D organ model corresponding to an observation target organ to be observed; a step of acquiring a camera video; a step of recognizing resection of the observation target organ from the camera video and outputting first resection information including whether the resection has or has not been completed at a first resection timing; and a step of performing display related to whether the resection has or has not been completed in the 3D organ model based on the first resection information.

According to the present disclosure, it is possible to display whether resection of a target to be resected has or has not been completed without requiring a user to take any time and effort.

### BRIEF DESCRIPTION OF THE DRAWINGS

FIG. 1 is a schematic diagram of a medical system.
FIG. 2 is an image diagram of a laparoscopic video and a 3D organ model.
FIG. 3A is a block diagram showing functions of a first resection information generation unit, and FIG. 3B is a block diagram showing functions of a display controller.
FIG. 4 is an explanatory diagram showing a resection cross section that has been resected.
FIG. 5A is an explanatory diagram showing blood vessels for which resection has or has not been completed using different colors, and FIG. 5B is an explanatory diagram showing blood vessels for which resection has or has not been completed using different mark shapes.
FIG. 6A is an explanatory diagram showing a lesion model for which resection has not been completed, and FIG. 6B is an explanatory diagram showing that a lesion model for which resection has been completed is hidden.
FIG. 7A is a block diagram showing functions of a second resection information generation unit in a case of using a 3D organ model, and FIG. 7B is a block diagram showing functions of a second resection information generation unit in a case of using resection plan information.
FIG. 8 is an explanatory diagram showing a blood vessel to be resected next.
FIG. 9 is an explanatory diagram showing a lesion to be resected next.
FIG. 10 is an explanatory diagram showing a blood vessel for which resection has been completed, a blood vessel for which resection has not been completed, or a blood vessel to be resected next.
FIG. 11 is a block diagram showing functions of a posture matrix estimation unit.
FIG. 12 is an explanatory diagram showing a three-dimensional coordinate system consisting of an X-axis, a Y-axis, and a Z-axis.
FIG. 13 is an explanatory diagram showing 3D organ models before and after display control based on a posture matrix.
FIG. 14 is a block diagram showing functions of a display controller in a case of using the posture matrix.
FIG. 15 is an explanatory diagram showing a blood vessel for which resection has been completed and a blood vessel for which resection has not been completed in the 3D organ models before and after the display control based on the posture matrix.
FIG. 16A is a block diagram showing functions of the first resection information generation unit in a case of using laparoscopic videos captured by a plurality of cameras, and FIG. 16B is a block diagram showing functions of the first resection information generation unit in a case of using an ultrasound video.
FIG. 17A is an explanatory diagram showing a case of outputting first resection information for each blood vessel, and FIG. 17B is an explanatory diagram showing a case of outputting first resection information for each lesion.
FIG. 18 is a flowchart showing a series of flows of performing display related to whether the resection has or has not been completed using a laparoscopic video.
FIG. 19A is a block diagram showing functions of the first resection information generation unit in a case of using a correspondence relationship between an intraoperative structure and a preoperative structure, and FIG. 19B is a block diagram showing functions of the first resection information generation unit in a case of using intraoperative and preoperative 3D organ models.
FIG. 20 is a block diagram showing functions of the first resection information generation unit in a case of using the posture matrix.
FIG. 21 is a block diagram showing functions of a resection alert unit.
FIG. 22 is a block diagram showing functions of a plan change detection unit and a resection plan information correction unit.

### DESCRIPTION OF THE PREFERRED EMBODIMENTS

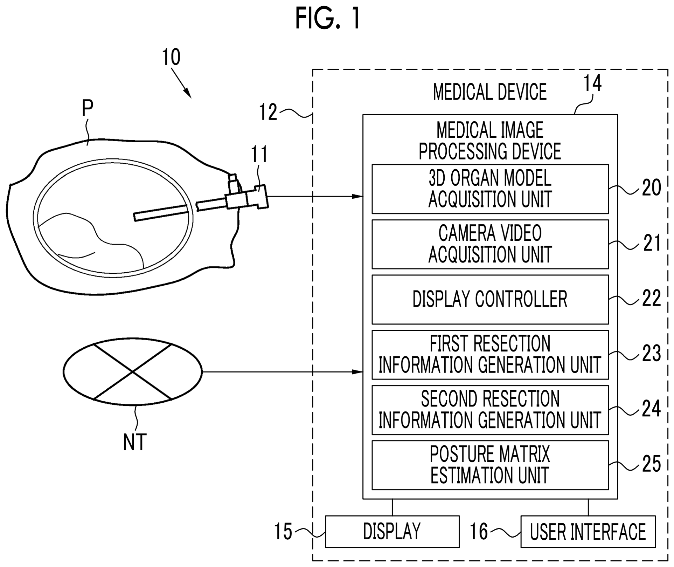

As shown in FIG. 1, a medical system 10 comprises a laparoscope 11 and a medical device 12. The laparoscope 11 captures an image of an inside of a body of a patient P and transmits a laparoscopic video obtained by the capturing to the medical device 12. The laparoscope 11 is also connected to a light source device (not shown), and illumination light from the light source device is supplied to the laparoscope 11.

The medical device 12 comprises a medical image processing device 14 configured by a computer such as a server, a display 15, and a user interface 16. In addition, the medical image processing device 14 is connected to a network NT. A picture archiving and communication system (PACS) or the like is connected to the network NT, and various image data and the like from the PACS are incorporated into the medical image processing device 14 via the network NT.

In the medical image processing device 14, a program for executing various types of processing is stored in a program memory (not shown). A central controller (not shown) configured by a processor executes the program in the program memory, whereby the medical image processing device 14 implements functions of a 3D organ model acquisition unit 20, a camera video acquisition unit 21, a display controller 22, a first resection information generation unit 23, a second resection information generation unit 24, and a posture matrix estimation unit 25.

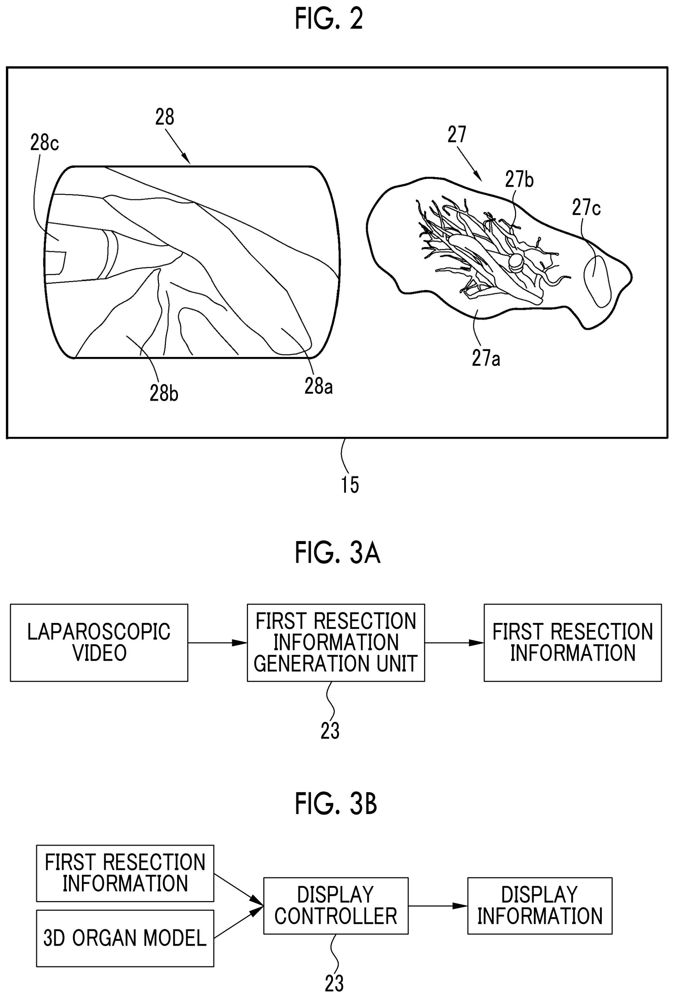

The 3D organ model acquisition unit 20 acquires a 3D organ model corresponding to an observation target organ to be observed. The 3D organ model is acquired from a 3D organ model image server (not shown) or the like via the network NT. The 3D organ model is a model extracted from a radiation image such as an X-ray image or a CT image, or an MRI image. As shown in FIG. 2, a 3D organ model 27 is configured by displaying a blood vessel, a lesion, or the like on the observation target organ. Specifically, in a case where the observation target organ is the liver, a model in which an internal blood vessel 27b or a lesion 27c of the liver is displayed on a liver 27a is displayed on the display 15. The display on the display 15 is controlled by the display controller 22. In addition to the liver, the observation target organ may be, for example, a kidney, a pancreas, a spleen, a uterus, a nerve, a lung, a bronchus, an intracranial blood vessel, or a prostate, and is not limited to the above organs and may be various other organs. In addition, the lesion is, for example, a mass, a tumor, or a cyst.

The camera video acquisition unit 21 acquires a camera video. In the present embodiment, the camera video is a laparoscopic video captured with the laparoscope 11. Specifically, as shown in FIG. 2, a laparoscopic video 28 includes a liver 28a, a structure 28b around the liver 28a, an ultrasound probe 28c that is one of various treatment tools, and the like. The laparoscopic video 28 is displayed in parallel with the 3D organ model 27 on the display 15. The display of the laparoscopic video 28 is controlled by the display controller 22. It is preferable that the camera video be a color video, and various medical videos other than the laparoscopic video may be used. In addition, the laparoscopic video 28 may be displayed alone on the display 15 without being displayed in parallel with the 3D organ model 27.

The first resection information generation unit 23 recognizes resection of the observation target organ from the laparoscopic video, and outputs first resection information including whether the resection has or has not been completed at a first resection timing. The first resection information generation unit 23 is configured by a learning model that has been trained using a laparoscopic video in which resection has been performed. As shown in FIG. 3A, the first resection information generation unit 23 outputs the first resection information in a case where the resection of the observation target organ is recognized in the input laparoscopic video. The resection information is information for identifying, on the 3D organ model, a blood vessel or a lesion for which resection has been completed. The resection information includes, for example, coordinate information of the 3D organ model, preliminary information (scheduled resection blood vessel, order of resection, and the like) set during preoperative planning, and branch information of the blood vessel. The output first resection information is input to the display controller 22. It is preferable that a timing at which the first resection information generation unit 23 recognizes the resection be set as the first resection timing. The term "timing" in the present embodiment includes the possibility that the timing of the start and end of the resection is shifted as long as the resection is performed at the same moment.

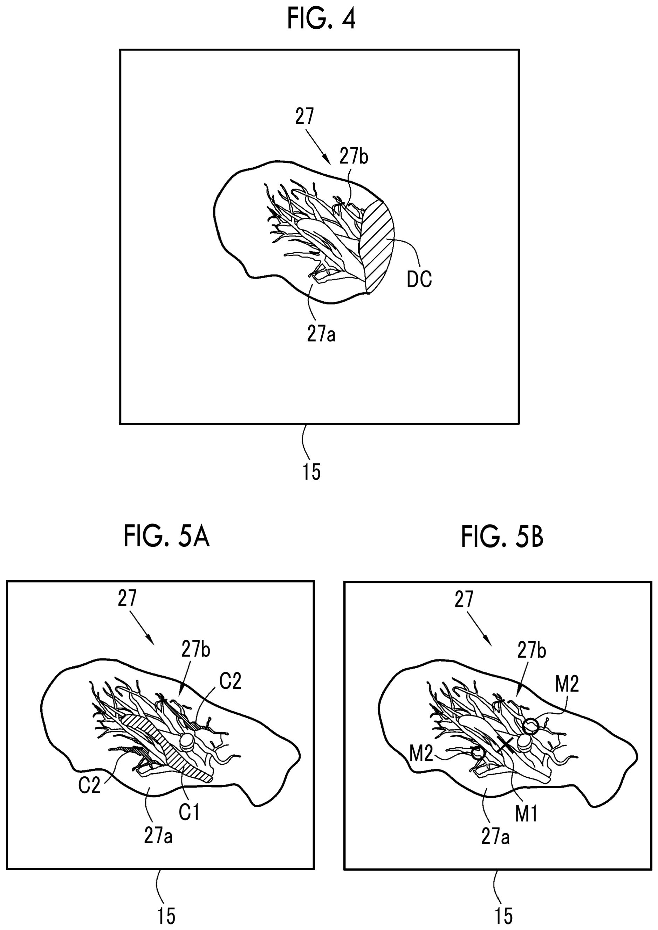

The display controller 22 performs display related to whether the resection has or has not been completed in the 3D organ model based on the first resection information. As shown in FIG. 3B, in response to the input of the first resection information and the 3D organ model, the display controller 22 outputs, to the display 15, information on the display related to whether the resection has or has not been completed in the 3D organ model. It is preferable that the display related to whether the resection has or has not been completed in the 3D organ model be display for distinguishing between completed resection and incomplete resection in the 3D organ model. For example, as shown in FIG. 4, of the liver 27a, a resection cross section that has been resected is displayed as a resection cross section DC in a predetermined color indicating the completed resection, and a non-resected portion is displayed as the incomplete resection by not changing the display.

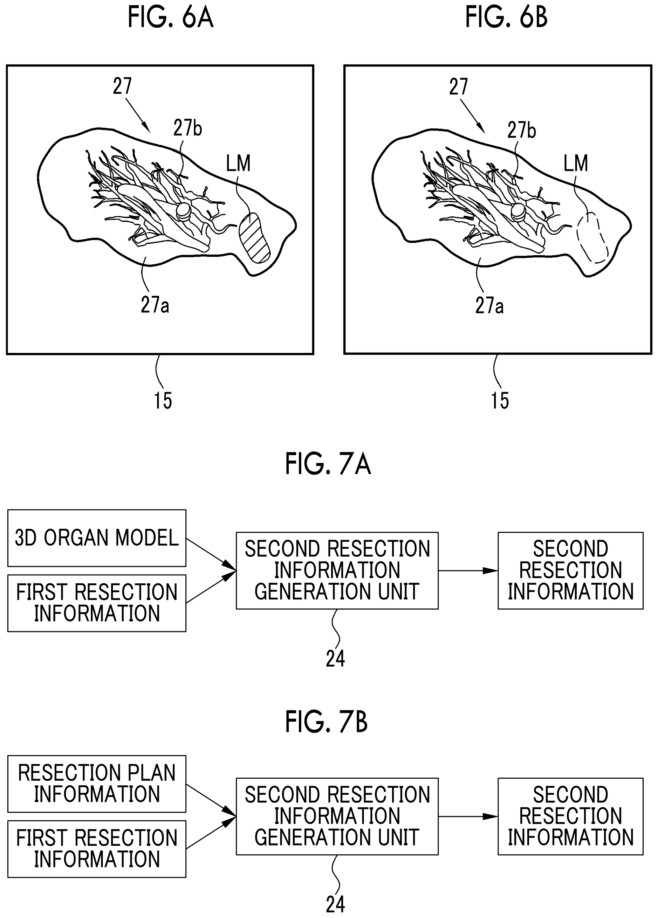

In addition, as shown in FIG. 5A, in the 3D organ model, among the blood vessels 27b included in the liver 27a, a resected blood vessel is displayed in a first color C1 indicating the completed resection, and a blood vessel that has not been resected but is scheduled to be resected is displayed in a second color C2 indicating the incomplete resection. In addition, as shown in FIG. 5B, for the blood vessel for which resection has been completed, a cross mark M1 indicating the completed resection may be displayed, and, for the blood vessel for which resection has not been completed, a circle mark M2 indicating the incomplete resection may be displayed. In addition, as shown in FIG. 6A, for a lesion included in the liver, a lesion that has not been resected is displayed as a lesion model LM indicating the incomplete resection. On the other hand, as shown in FIG. 6B, in a case where the lesion has been resected, the lesion model is hidden and the completion of resection is displayed (in FIG. 6B, the hidden state is represented by a dotted line). In this case, the lesion may be displayed in a faint manner by reducing the contrast with the surrounding area instead of completely hiding the lesion.

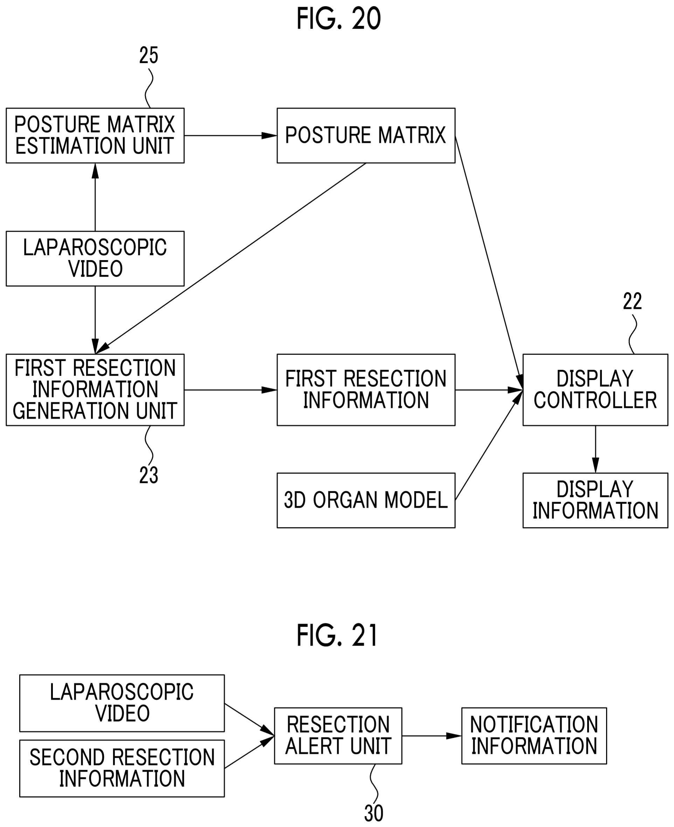

The second resection information generation unit 24 estimates a scheduled resection target that is scheduled to be resected at a second resection timing after the first resection timing from the 3D organ model and the first resection information, and outputs the scheduled resection target as second resection information. The second resection information generation unit 24 is configured by a learning model that has been trained using a 3D organ model and a laparoscopic video in which resection has been performed. As shown in FIG. 7A, the second resection information generation unit 24 estimates the scheduled resection target that is scheduled to be resected at the second resection timing after the first resection timing from the input 3D organ model and the input first resection information. Then, the second resection information generation unit 24 outputs the estimated scheduled resection target as the second resection information. The second resection timing may be a resection step performed next to the first resection timing, or may be a timing after a plurality of resection steps have been performed from the first resection timing. It is preferable that the second timing be set as appropriate by a user operation. In addition, the second resection information may be generated based on correspondence information (see FIG. 20) calculated by an intraoperative/preoperative correspondence relationship calculation unit 23g to be described below in addition to the first resection information.

As shown in FIG. 7B, the second resection information generation unit 24 may estimate a scheduled resection target that is scheduled to be resected at the second resection timing from predetermined resection plan information and the first resection information, and outputs the scheduled resection target as second resection information. It is preferable that the resection plan information include at least anatomical structure information for identifying a position, a size, a range, or an anatomical structure of the scheduled resection target in the observation target organ, and information on an order in which the scheduled resection target is resected.

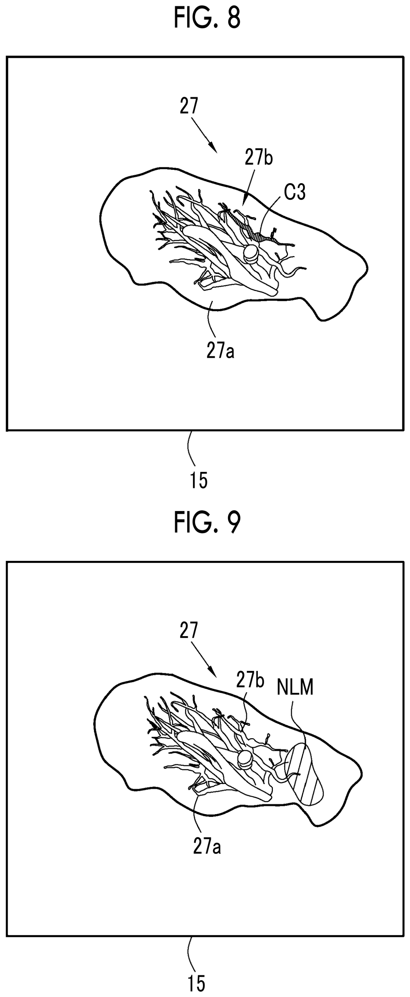

The display controller 22 performs display related to the scheduled resection target in the 3D organ model based on the second resection information. It is preferable that the display related to the scheduled resection target in the 3D organ model be display for distinguishing between the scheduled resection target and other portions in the observation target organ. For example, as shown in FIG. 8, in the 3D organ model 27, among blood vessels included in the liver 27a, a blood vessel to be resected next is displayed in a third color C3 indicating the scheduled resection target, and the other blood vessels are displayed in colors other than the third color C3. For the blood vessel that is the scheduled resection target, a scheduled resection mark of a predetermined shape may be displayed. In addition, as shown in FIG. 9, in the 3D organ model 27, for a lesion included in the liver 27a, a lesion to be resected next is displayed as a next resection lesion model NLM indicating the scheduled resection target.

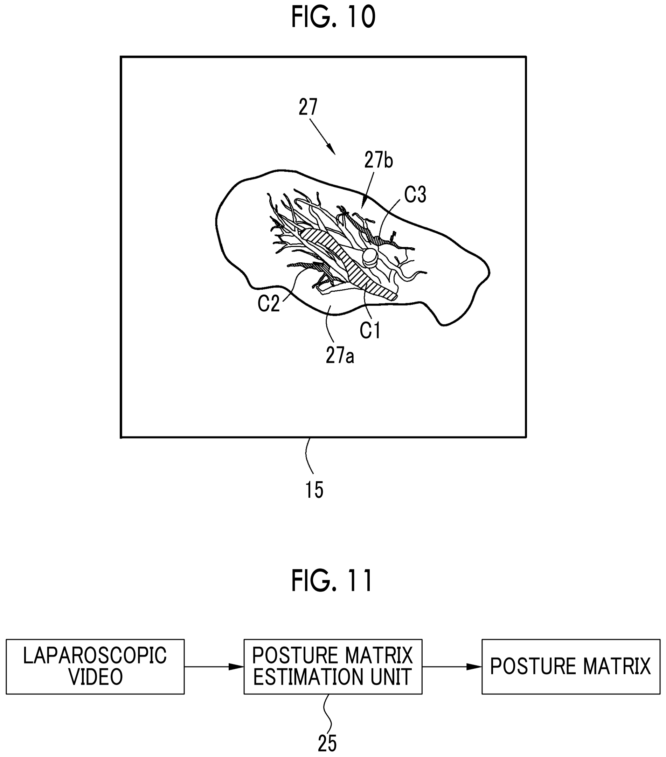

The display controller 22 may perform both the display related to whether the resection has or has not been completed in the 3D organ model and the display related to the scheduled resection target based on the second resection information in addition to the first resection information. In addition, as shown in FIG. 10, in the 3D organ model 27, among the blood vessels included in the liver 27a, a blood vessel to be resected next is displayed in a third color C3 indicating the next scheduled resection target, and the other blood vessels are displayed in colors other than the third color C3. In addition, a blood vessel that has already been resected is displayed in a first color C1, and a blood vessel that has not yet been resected but is scheduled to be resected is displayed in a second color C2. In this way, by displaying the blood vessels that are the scheduled resection targets in different colors, depending on whether they are scheduled to be resected next or other blood vessels, it is possible to understand how many blood vessels remain to be resected.

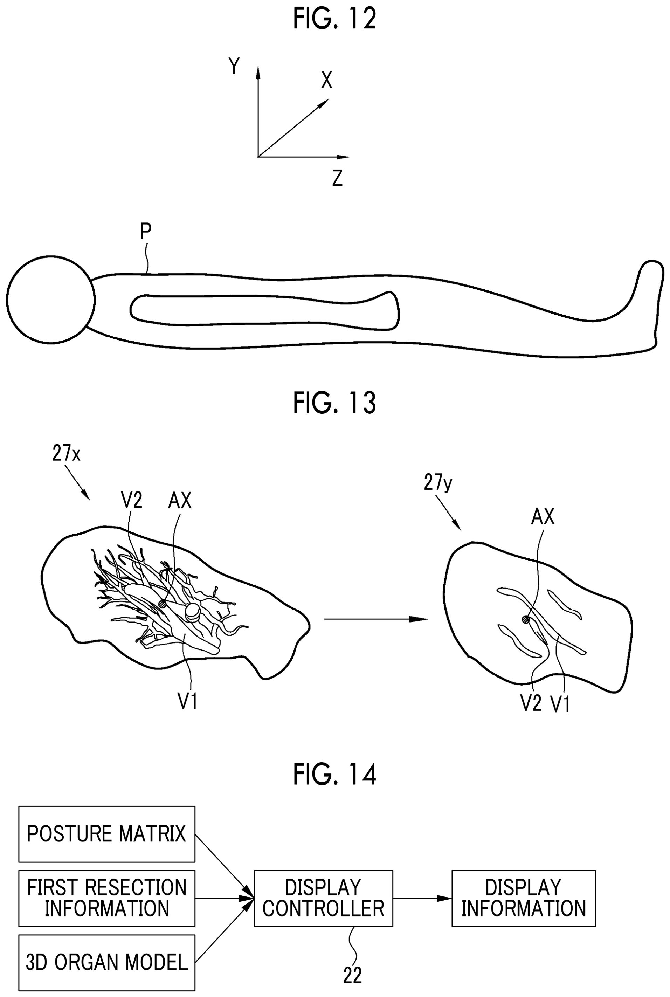

The posture matrix estimation unit 25 estimates, from the laparoscopic video, a pose transformation matrix (a posture matrix) in a Viewer coordinate system that displays the 3D organ model, the posture matrix representing a spatial pose (a posture) of the observation target organ. The posture matrix estimation unit 25 uses a learning model that has been trained using the laparoscopic video and a ground-truth posture matrix for the laparoscopic video as inputs. As shown in FIG. 11, in a case where the laparoscopic video is input to the posture matrix estimation unit 25, the posture matrix is output from the posture matrix estimation unit 25. As shown in FIG. 12, the Viewer coordinate system is represented by three axes of an X-axis, a Y-axis, and a Z-axis. The X-axis is represented by a positive value that is zero on a right side of the patient P and that increases toward a left side. The Y-axis is represented by a positive value that is zero on a ventral side of the patient P and that increases toward a dorsal side. The Z-axis is represented by a positive value that is zero on a head side of the patient P and that increases toward a foot side. The three-dimensional coordinate system may be a polar coordinate system represented by a radius and a polar angle in addition to an orthogonal coordinate system such as an X-axis, a Y-axis, and a Z-axis, and is not particularly limited. In addition, instead of the learning model, a plurality of models such as a model that extracts a feature amount from the laparoscopic video and a model that estimates the posture matrix from the feature amount may be used to estimate the posture matrix.

The display controller 22 performs display related to the posture of the observation target organ based on the posture matrix. Specifically, as shown in FIG. 13, a 3D organ model 27x represents a model before display control based on the posture matrix, and a 3D organ model 27y represents a model after display control based on the posture matrix. The 3D organ model 27y is a model that is rotated relative to the 3D organ model by a predetermined angle about a rotation axis AX. As a result, in the 3D organ model 27x, a blood vessel V2 under a thick blood vessel V1 is not visible in terms of its running path, whereas, in the 3D organ model 27y rotated by the predetermined angle, the blood vessel V2 under the thick blood vessel V1 becomes visible, enabling confirmation of the running state of the blood vessel V2. The rotation axis AX can be set arbitrarily in addition to being based on the posture matrix. For example, a user's line of sight (line connecting the center of gravity of the observation target organ and the center of the field of view of the laparoscope 11) may be used as the rotation axis.

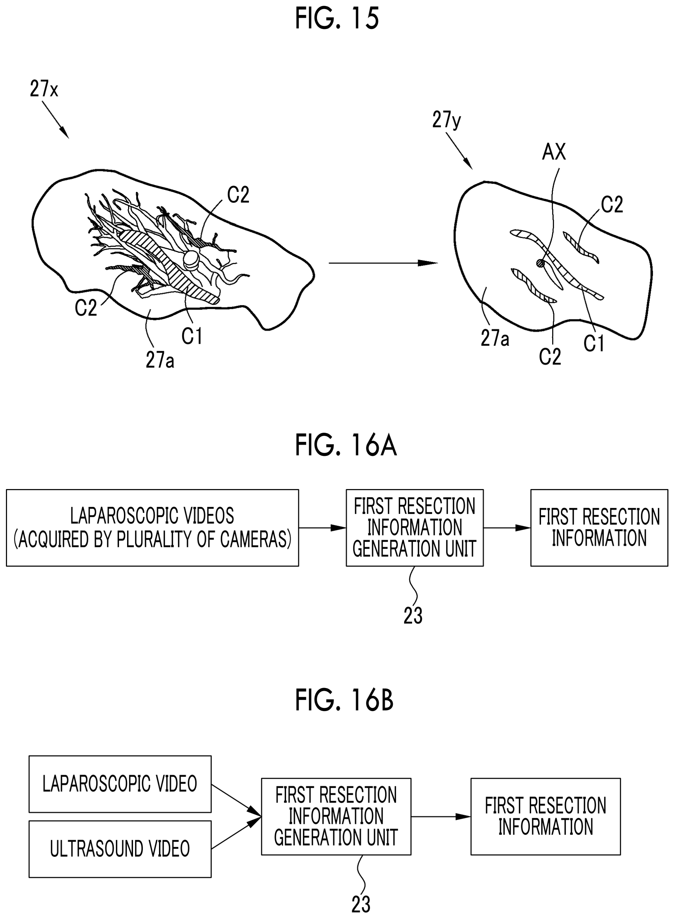

The display controller 22 may perform both the display related to whether the resection has or has not been completed in the 3D organ model and the display related to the posture of the observation target organ based on the posture matrix in addition to the first resection information. In this case, as shown in FIG. 14, the display controller 22 outputs information for the display related to whether the resection has or has not been completed in the 3D organ model and the display related to the posture of the observation target organ in response to the input of the posture matrix, the first resection information, and the 3D organ model. For example, as shown in FIG. 15, in a 3D organ model 27x before display control based on the posture matrix, a blood vessel for which resection has been completed is displayed in a first color C1, and a blood vessel for which resection has not been completed is displayed in a second color C2. Then, in a 3D organ model 27y rotated by a predetermined angle after display control based on the posture matrix, a blood vessel for which resection has been completed is displayed in a first color C1, and a blood vessel for which resection has not been completed is displayed in a second color C2. In order to avoid making it difficult to see in a case where the posture information is updated during the resection, the first resection information may include a non-resection status, a resection-in-progress status, or a completed resection status, and, in a case of the resection-in-progress status, a value of the posture matrix may be set to a fixed value until the completed resection is reached (by setting the fixed value, the posture of the observation target organ is maintained during the resection).

As shown in FIG. 16A, the first resection information generation unit 23 may output the first resection information based on videos captured by a plurality of cameras provided on the laparoscope 11 as the laparoscopic video. In addition, as shown in FIG. 16B, the first resection information generation unit 23 may output the first resection information based on the ultrasound video in addition to the laparoscopic video. The ultrasound video is preferably a video obtained by imaging the observation organ with the ultrasound probe 28c (see FIG. 2). Any one or more of the laparoscopic video, the stereo video, the 3D organ model, or the ultrasound video may be input to the first resection information generation unit 23.

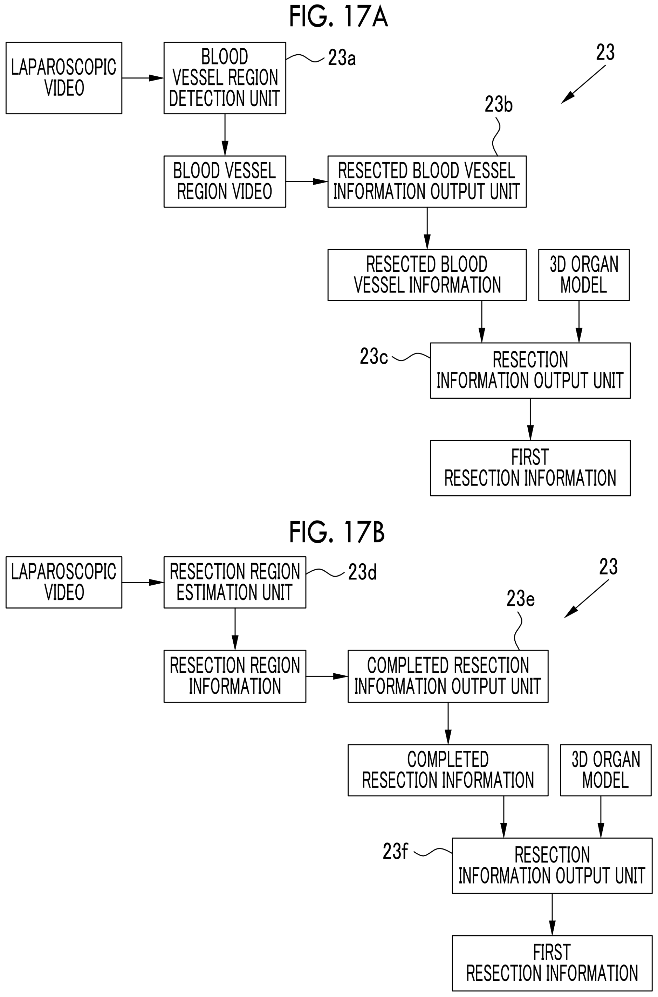

The first resection information generation unit 23 directly outputs the first resection information from the laparoscopic video, but the first resection information may be output by another method. For example, in a case of outputting the first resection information for each blood vessel, as shown in FIG. 17A, the first resection information generation unit 23 is configured by a blood vessel region detection unit 23a, a resected blood vessel information output unit 23b, and a resection information output unit 23c. The blood vessel region detection unit 23a detects a blood vessel region from the laparoscopic video and outputs a blood vessel region video. The resected blood vessel information output unit 23b outputs resected blood vessel information as information on a resected blood vessel from the blood vessel region video. Then, the resection information output unit 23c acquires information on the completed resection and the incomplete resection for the blood vessel to be resected from the resected blood vessel information and the 3D organ model, and outputs the first resection information.

In addition, in a case of outputting the first resection information for each lesion, as shown in FIG. 17B, the first resection information generation unit 23 is configured by a resection region estimation unit 23d, a completed resection information output unit 23e, and a resection information output unit 23f. The resection region estimation unit 23d estimates a resection region from the laparoscopic video and outputs resection region information. The completed resection information output unit 23e outputs completed resection information as information on the completed resection for the resection region from the resection region information. Then, the resection information output unit 23f acquires information on the completed resection and the incomplete resection for the region to be resected, such as the lesion, from the completed resection information and the 3D organ model, and outputs the first resection information.

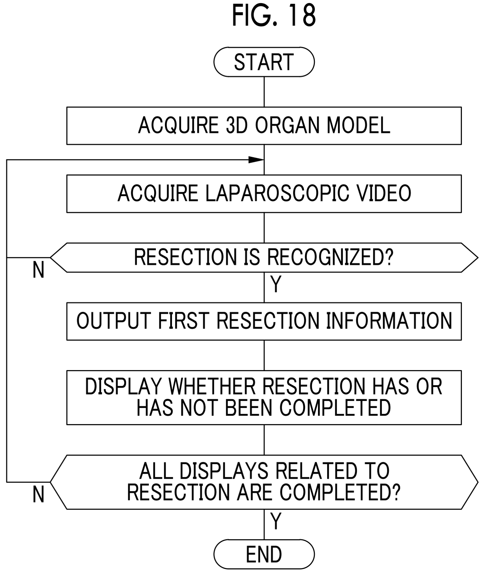

Next, a series of flows of performing the display related to whether the resection of the resection target has or has not been completed using the laparoscopic video will be described with reference to a flowchart of FIG. 18. The 3D organ model acquisition unit 20 acquires the 3D organ model 27 corresponding to the observation target organ to be observed via the network NT. The camera video acquisition unit 21 acquires the laparoscopic video 28 obtained by the laparoscope 11. In a case where the first resection information generation unit 23 recognizes resection of the observation target organ from the laparoscopic video, the first resection information generation unit 23 outputs the first resection information including whether the resection has or has not been completed at the first resection timing. The display controller 22 performs, on the display 15, display related to whether the resection has or has not been completed in the 3D organ model 27 based on the first resection information. The series of flows after acquisition of the laparoscopic video is repeatedly performed until all displays related to the resection are completed.

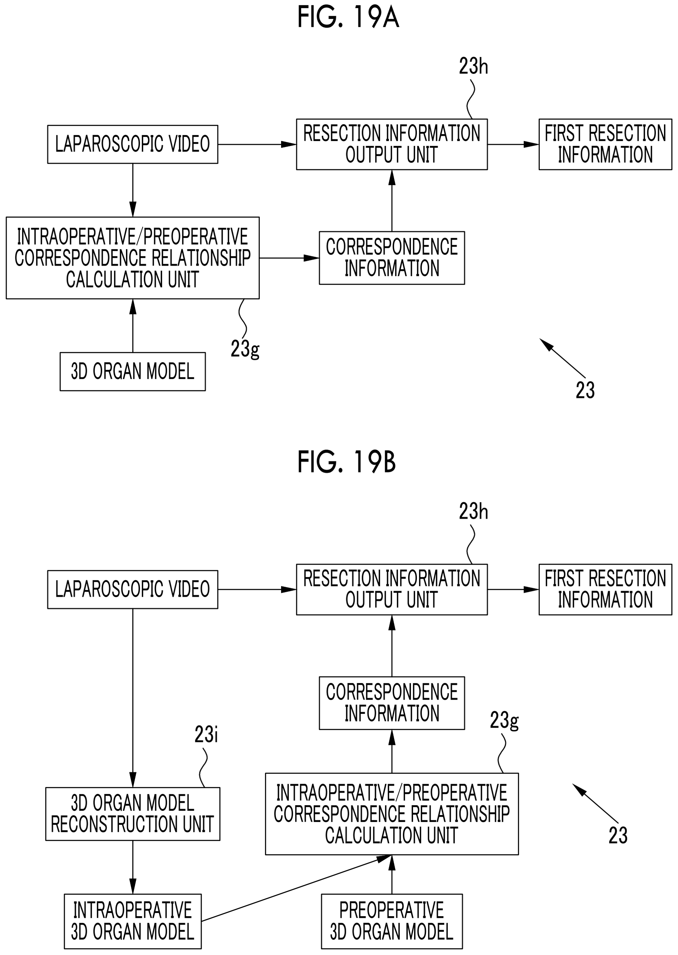

In the above-described embodiment, the first resection information is directly output from the laparoscopic video, but the first resection information may be output by another method. As shown in FIG. 19A, the first resection information generation unit 23 may be divided into the intraoperative/preoperative correspondence relationship calculation unit 23g and a resection information output unit 23h. In this case, the intraoperative/preoperative correspondence relationship calculation unit 23g outputs correspondence information indicating a correspondence relationship between an intraoperative structure and a preoperative structure from an intraoperative laparoscopic video (during observation of the observation target organ) and a preoperative 3D organ model (during non-observation of the observation target organ). The structure includes an organ, a blood vessel, a lesion, and the like. The resection information output unit 23h outputs the first resection information from the laparoscopic video and the correspondence information. As described above, by using the correspondence information, it is possible to improve the accuracy of identifying the structure for which resection has or has not been completed. As the calculation processing by the intraoperative/preoperative correspondence relationship calculation unit 23g, for example, a method of outputting a virtual cross section from the preoperative 3D organ model, comparing the virtual cross section with the laparoscopic video, and calculating the correspondence relationship is used. In addition, the term "intraoperative" refers to a case where the observation target organ is being observed with the laparoscopic video and surgery or the like is being performed, and the term "preoperative" refers to a case where observation or surgery using the laparoscope 11 is not being performed.

As shown in FIG. 19B, the first resection information generation unit 23 may be divided into the intraoperative/preoperative correspondence relationship calculation unit 23g, the resection information output unit 23h, and a 3D organ model reconstruction unit 23i. In this case, the 3D organ model reconstruction unit 23i reconstructs the intraoperative 3D organ model in response to the input of the laparoscopic video. Then, the intraoperative/preoperative correspondence relationship calculation unit 23g outputs the correspondence information from the preoperative 3D organ model and the intraoperative 3D organ model. As a result, it is possible to further improve the accuracy of identifying the structure for which resection has or has not been completed. As shown in FIG. 20, the first resection information generation unit 23 may output the first resection information in response to the input of the posture matrix obtained by the posture matrix estimation unit 25 in addition to the laparoscopic video. As described above, the posture matrix is useful information for determining which lesion or blood vessel is to be resected, so that, by using the posture matrix as well, the accuracy of identifying the structure for which resection has or has not been completed can be improved. The 3D organ model reconstruction unit 23i may reconstruct the intraoperative 3D organ model in response to the input of the ultrasound video.

In the above-described embodiment, as shown in FIG. 21, a resection alert unit 30 may issue an alert for the next second resection timing. The resection alert unit 30 outputs notification information in a case where an attempt is made to resect a lesion or a blood vessel other than the lesion or the blood vessel to be resected (a lesion or a blood vessel that should not be resected) in response to the input of the laparoscopic video and the second resection information. The medical device 12 issues an alert to the user based on the notification information. The notification method is, for example, an alert display on the display 15 or a notification by a sound.

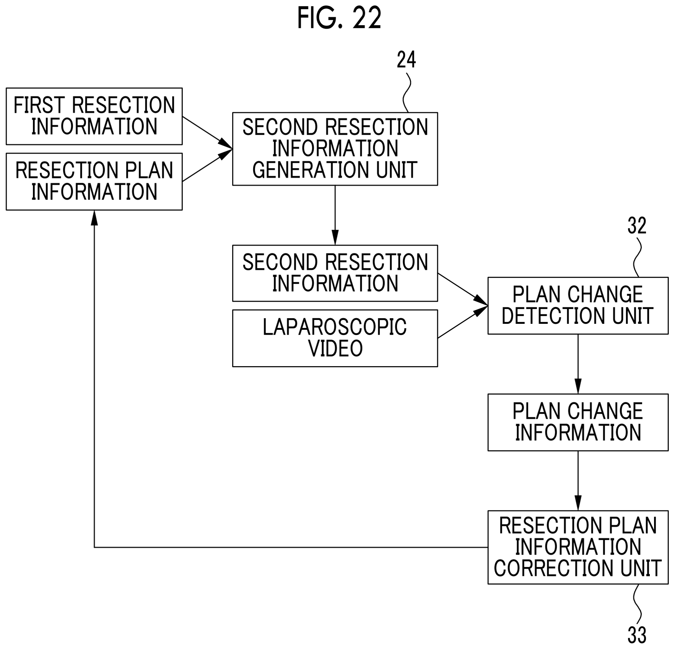

In the above-described embodiment, in a case of generating the second resection information by using the resection plan information, changes in the resection plan information may be detected and corrected in consideration of changes during surgery. In this case, as shown in FIG. 22, a plan change detection unit 32 and a resection plan information correction unit 33 are used. The plan change detection unit 32 outputs plan change information indicating whether or not the resection plan information has been changed during the surgery in response to the input of the second resection information and the laparoscopic video. The resection plan information correction unit 33 corrects the resection plan information based on the plan change information. In a case where the resection plan is changed in the plan change information, the resection plan information is corrected in accordance with the current situation.

In the present embodiment, each process of the display controller 22, the first resection information generation unit 23, the second resection information generation unit 24, the posture matrix estimation unit 25, the blood vessel region detection unit 23a, the resected blood vessel information output unit 23b, the resection information output unit 23c, the resection region estimation unit 23d, the completed resection information output unit 23e, the resection information output unit 23f, the intraoperative/preoperative correspondence relationship calculation unit 23g, the resection information output unit 23h, the 3D organ model reconstruction unit 23i, the resection alert unit 30, the plan change detection unit 32, and the resection plan information correction unit 33 is executed by any computer. In addition, any computer may execute the processing using a processor, a program, or a combination thereof. Any computer may be a general-purpose computer, a computer for a specific use, a system such as a workstation, or other hardware elements capable of executing a program. It is preferable that the blood vessel region detection unit 23a, the resected blood vessel information output unit 23b, the resection information output unit 23c, the resection region estimation unit 23d, the completed resection information output unit 23e, the resection information output unit 23f, the intraoperative/preoperative correspondence relationship calculation unit 23g, the resection information output unit 23h, the 3D organ model reconstruction unit 23i, the resection alert unit 30, the plan change detection unit 32, and the resection plan information correction unit 33 use a learning model as with the first resection information generation unit 23 and the like.

The processor may be configured by one or more pieces of hardware, and the type of hardware is not limited. For example, the processor may be configured by a programmable logic device such as a central processing unit (CPU), a micro processing unit (MPU), or a field programmable gate array (FPGA), a dedicated circuit for executing specific processing such as an application specific integrated circuit (ASIC), or hardware such as a graphics processing unit (GPU) or a neural processing unit (NPU). In addition, the processor has each unit or each means that executes various types of processing in the present embodiment. In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each processing executed by the processor is not limited to the above order and may be changed as appropriate. The hardware is configured by an electric circuit (circuitry) in which circuit elements such as semiconductor elements are combined.

Further, the present embodiment may be realized by hardware, software, firmware, microcode, or a combination thereof. Software, firmware, and microcode are configured by a program. In addition, the program may be, for example, a program module group, and each function thereof may be realized by a processor configured to execute each function. The program may be a program code or a plurality of code segments stored in one or a plurality of non-transitory computer-readable media (for example, a recording medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

### Explanation of References

10: medical system
11: laparoscope
12: medical device
14: medical image processing device
15: display
16: user interface
20: 3D organ model acquisition unit
21: camera video acquisition unit
22: display controller
23: first resection information generation unit
23a: blood vessel region detection unit
23b: resected blood vessel information output unit
23c: resection information output unit
23d: resection region estimation unit
23e: completed resection information output unit
23f: resection information output unit
23g: intraoperative/preoperative correspondence relationship calculation unit
23h: resection information output unit
23i: 3D organ model reconstruction unit
24: second resection information generation unit
25: posture matrix estimation unit
27, 27x, 27y: 3D organ model
27a: liver
27b: blood vessel
27c: lesion
28: laparoscopic video
28a: liver
28b: structure
28c: ultrasound probe
30: resection alert unit
32: plan change detection unit
33: resection plan information correction unit
P: patient
NT: network
DC: resection cross section
C1: first color
C2: second color
C3: third color
LM: lesion model
NLM: next resection lesion model
V1, V2: blood vessel
AX: rotation axis

## Claims

1. A medical device (12) comprising:
a processor,
wherein the processor
acquires a 3D organ model (27) corresponding to an observation target organ to be observed,
acquires a camera video,
recognizes resection of the observation target organ (27a) from the camera video and outputs first resection information including whether the resection has or has not been completed at a first resection timing, and
performs display related to whether the resection has or has not been completed in the 3D organ model (27) based on the first resection information.

2. The medical device according to claim 1,
wherein the processor
estimates a scheduled resection target that is scheduled to be resected at a second resection timing after the first resection timing from the 3D organ model (27) and the first resection information, and outputs the scheduled resection target as second resection information, and
performs, based on the second resection information in addition to the first resection information, the display related to whether the resection has or has not been completed in the 3D organ model and display related to the scheduled resection target, or performs, based on the second resection information instead of the first resection information, display related to the scheduled resection target in the 3D organ model.

3. The medical device according to claim 1,
wherein the processor
estimates a scheduled resection target that is scheduled to be resected at a second resection timing after the first resection timing from predetermined resection plan information and the first resection information, and outputs the scheduled resection target as second resection information, and
performs, based on the second resection information in addition to the first resection information, the display related to whether the resection has or has not been completed in the 3D organ model and display related to the scheduled resection target, or performs, based on the second resection information instead of the first resection information, display related to the scheduled resection target in the 3D organ model (27).

4. The medical device according to claim 1,
wherein the processor
estimates, from the camera video, a pose transformation matrix in a Viewer coordinate system that displays the 3D organ model (27), the pose transformation matrix representing a spatial pose of the observation target organ, and
performs, based on the pose transformation matrix in addition to the first resection information, the display related to whether the resection has or has not been completed in the 3D organ model and display related to the spatial pose of the observation target organ.

5. The medical device according to claim 1,
wherein the display related to whether the resection has or has not been completed in the 3D organ model (27) is display for distinguishing between completed resection and incomplete resection in the 3D organ model.

6. The medical device according to claim 5,
wherein, of the observation target organ (27a), a resection cross section that has been resected is displayed as the completed resection, and a non-resected portion is displayed as the incomplete resection.

7. The medical device according to claim 5,
wherein, among blood vessels included in the observation target organ (27a), a resected blood vessel (27b) is displayed as the completed resection, and a blood vessel that has not been resected but is scheduled to be resected is displayed as the incomplete resection.

8. The medical device according to claim 5,
wherein, for a lesion (27c) included in the observation target organ, a lesion that has not been resected is displayed as the incomplete resection, and a resected lesion is displayed as the completed resection.

9. The medical device according to claim 2,
wherein the display related to the scheduled resection target in the 3D organ model (27) is display for distinguishing between the scheduled resection target and other portions in the observation target organ.

10. The medical device according to claim 9,
wherein a resection cross section to be resected at the second resection timing in the observation target organ is displayed as the scheduled resection target.

11. The medical device according to claim 9,
wherein a blood vessel or a lesion to be resected at the second resection timing among blood vessels or lesions included in the observation target organ is displayed as the scheduled resection target.

12. The medical device according to claim 1,
wherein the camera video is obtained from videos from a plurality of cameras.

13. The medical device according to claim 1,
wherein the processor outputs the first resection information based on an ultrasound video in addition to the camera video.

14. The medical device according to claim 1,
wherein the processor outputs the first resection information for each blood vessel or each lesion included in the observation target organ.

15. The medical device according to claim 1,
wherein the processor outputs the first resection information based on correspondence information indicating a correspondence relationship between an anatomical structure in the camera video and a structure in the 3D organ model in addition to the camera video.

16. The medical device according to claim 15,
wherein the processor reconstructs an intraoperative 3D organ model (27) of the observation target organ (27a) from the camera video or an ultrasound video, and
the correspondence relationship is calculated from the intraoperative 3D organ model and the 3D organ model.

17. The medical device according to claim 4,
wherein the processor outputs the first resection information based on the pose transformation matrix in addition to the camera video.

18. The medical device according to claim 2 or 3,
wherein the processor outputs notification information based on the camera video and the second resection information.

19. The medical device according to claim 3,
wherein the processor outputs plan change information based on the camera video and the second resection information, and corrects the resection plan information based on the plan change information.

20. An operation method of a medical device (12), the operation method comprising:
a step of acquiring a 3D organ model (27) corresponding to an observation target organ (27a) to be observed;
a step of acquiring a camera video;
a step of recognizing resection of the observation target organ from the camera video and outputting first resection information including whether the resection has or has not been completed at a first resection timing; and
a step of performing display related to whether the resection has or has not been completed in the 3D organ model based on the first resection information.
